(19) Europäisches Patentamt
European Patent Office
Office européen des brevets

(11) Publication number: **0 068 038**
**B1**

(12) # EUROPEAN PATENT SPECIFICATION

(45) Date of publication of patent specification: **25.09.85**

(51) Int. Cl.$^4$: **C 07 D 309/30**, C 07 C 59/56, A 61 K 31/365, A 61 K 31/19

(21) Application number: **81105013.7**

(22) Date of filing: **29.06.81**

(54) **(+)-(4R,6S)-(E)-6-(2-(4'-fluoro-3,3',5-trimethyl-(1,1'-biphenyl)-2-yl)ethenyl)-3,4,5,6-tetrahydro-4-hydroxy-2H-pyran-2-one, a process for preparing and a pharmaceutical composition containing the same.**

(43) Date of publication of application:
**05.01.83 Bulletin 83/01**

(45) Publication of the grant of the patent:
**25.09.85 Bulletin 85/39**

(84) Designated Contracting States:
**AT BE CH DE FR GB IT LI LU NL SE**

(56) References cited:
**EP-A-0 010 299**
**EP-A-0 010 951**
**EP-A-0 024 348**

**The file contains technical information submitted after the application was filed and *not included in this specification***

(73) Proprietor: **MERCK & CO. INC.**
**126, East Lincoln Avenue P.O. Box 2000**
**Rahway New Jersey 07065 (US)**

(72) Inventor: **Willard, Alvin K.**
**8 Maplewood Lane**
**Wilmington Delaware 19810 (US)**
Inventor: **Novello, Frederick C.**
**786 Bair Road**
**Berwyn Pennsylvania 19312 (US)**
Inventor: **Hoffmann, William F.**
**740 Weikel Road**
**Lansdale Pennsylvania 19446 (US)**
Inventor: **Cragoe, Edward J., Jr.**
**2211 Oak Terrace Drive**
**Lansdale Pennsylvania 19446 (US)**

(74) Representative: **Abitz, Walter, Dr.-Ing. et al**
**Abitz, Morf, Gritschneder P.O. Box 86 01 09**
**D-8000 München 86 (DE)**

**EP 0 068 038 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Summary of the Invention

This invention relates to a hypocholesterolemic and hypolipemic compound which is a member of the class of compounds having the structure (I)

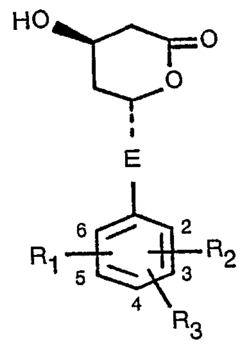

I

and the corresponding dihydroxy acid resulting from the hydrolytic opening of the lactone ring, and pharmaceutically acceptable salts of said acid. Specifically, it is the compound of structure I wherein E is *trans*—CH=CH—, $R_1$ is 6-(4′-fluoro-3′-methyl-phenyl, $R_2$ is 2-methyl, and $R_3$ is 4-methyl.

Background of the Invention

Recently, Endo et al, reported (U.S. Letters Patent 4,049,495, Patent 4,137,322 and Patent 3,983,140) the production of a fermentation product which was quite active in the inhibition of cholesterol biosynthesis. This natural product, now called compactin, was reported by Brown et al., (*J. Chem. Soc. Perkin I,* 1165 (1976)) to have a complex mevalonolactone structure.

Recent U.S. Patents 4,198,425 and 4,255,444 disclose a group of synthetic compounds of the generic formula II

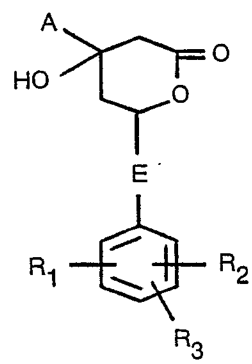

II

in which A is $—CH_3$ and hydrogen respectively, E represents a direct bond, a $C_{1-3}$ alkylene bridge or a vinylene bridge and the R groups represent a variety of substituents.

A patent application of Merck & Co., Inc., E.P. publication 0 024 348, discloses a similar series of synthetic compounds in which A is hydrogen and in which the lactone ring has a (4R)-*trans* configuration, the isomer which demonstrates all of the antihypercholesterolemic activity of the unresolved, *cis-trans* lactone mixture.

Now, with the present invention there is provided the compound, (+)-(4R, 6S)-(E)-6-[2-(3,3′,5-trimethyl-4′-fluoro-[1,1′-biphenyl]-2-yl)ethenyl]-3,4,5,6-tetrahydro-4-hydroxy-2H-pyran-2-one, which has an unexpectedly high order of efficacy relative to other members of the genus of which it is a member.

Detailed description of the Invention

This invention relates to a hypocholesterolemic and hypolipemic compound having the structure III.

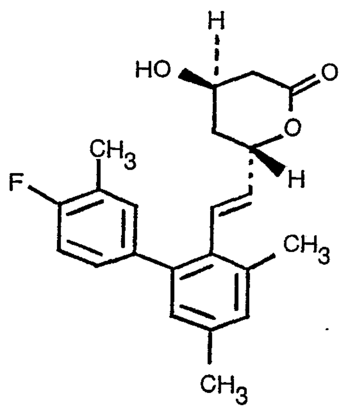

III

or the corresponding dihydroxy acid resulting from the hydrolytic opening of the lactone ring, or a pharmaceutically acceptable salt of the acid, the compound being the enantiomer with a 4 (*R*) configuration in the tetrahydropyran moiety of the *trans* racemate shown in the structures I and III.

The designation 4 (*R*) with respect to this compound indicates that the absolute configuration in space at the 4-carbon of the tetrahydropyranone ring is believed to be the Rectus (R) series, and it is dextrorotatory, and it is an unexpectedly potent inhibitor of cholesterol biosynthesis, surpassing the inhibitory activity of compactin.

The cited U.S. Patents 4,198,425 and 4,255,444 show no recognition of the stereochemistry of compounds II, let alone the fact that an unexpectedly large improvement in the activity would result from the separation of the *cis* and *trans* racemates and the latter's resolution, especially when the preferred 2,4,6-trisubstitution occurs in the phenyl ring. However, E.P. publication 0 024 348 discloses that the 4 (*R*) enantiomers of the *trans* racemates corresponding to formula I specifically inhibit with high potency the activity of 3-hydroxy-3-methylglutaryl-coenzyme A reductase, which is known to be the enzyme involved in the rate limiting step in the process of cholesterol biosynthesis.

The compound III of this invention is the most efficacious member of the latter series. The inhibitory activity of these compounds for the biosynthesis of cholesterol has been measured by two methods. The experimental method A of U.S. 4,198,425 was the *in vitro* method of H. J. Knauss, et al., *J. Biol. Chem., 234,* 2835 (1959) and the activity was expressed as the molar concentration $IC_{50}$ (M) necessary for the inhibition of 50% of the enzymatic activity. The experimental method B of U.S. 4,198,425 was the method of A. A. Kandutsch, et al., *J. Biol. Chem., 248,* 8403 (1973) for measuring $^{14}C$-cholesterol biosynthesis from acetic acid-$^{14}C$ in mouse L cells. The activity is expressed for inhibition of 50% of the biosynthesis of cholesterol.

The results obtained in these two asays for the compounds of Structure II, as reported in the cited U.S. patents, show $IC_{50}$ values of $10^{-4}$ to $10^{-6}$ in both tests. The smallest 50% effective dose cited is about $4 \times 10^{-6}$, whereas the value for compactin, in the same tests, is about $0.8 \times 10^{-8}$.

E.P. publication 0 024 348 shows that the inhibitory potency is greatly increased by separation of isomers especially when this is combined with optimal selection of a 2,4,6-arrangement of $R_1$, $R_2$ and $R_3$ in the phenyl ring of structure II and especially when A is hydrogen and E is *trans*-CH=CH—. Thus the (+) *trans* enantiomer of 6-[2-(2,4-dichloro-6-(phenylmethoxy)phenyl)ethyl]-3,4,5,6-tetrahydro-4-hydroxy-2H-pyran-2-one gives an $IC_{50}$ of $6.8 \times 10^{-8}$ in the test by method A. An even more potent compound is the (+) *trans* enantiomer of (E)-6-[2-(3,5-dichloro-4′-fluoro[1,1′-biphenyl]-2-yl)ethenyl]-3,4,5,6-tetrahydro-4-hydroxy-2H-pyran-2-one and the compound of the present invention, the (+)-trans enantiomer of (E)-6-[2-(4′-fluoro-3,3′, 5-trimethyl[1,1′-biphenyl]-2-yl)ethenyl]-3,4,5,6-tetrahydro-4-hydroxy-2H-pyran-2-one, is several times more potent.

The compounds were tested as the sodium salts of their corresponding dihydroxy acid forms. The compound of this invention has the added advantage of having no chloro substituents. Although the metabolism of the compound is not completely known it is advantageous not to have a compound that could be metabolized to a polychlorobiphenyl (PCB), a class of substances known to be involved in carcinogenesis.

The Flow Sheet shows the synthesis of the 4 (*R*) *trans* lactone of this invention. The number of each step in the Flow Sheet corresponds with the number of each reaction description and each step of Example 1 which follow. Steps 5 through 9 represent the novel process of this invention.

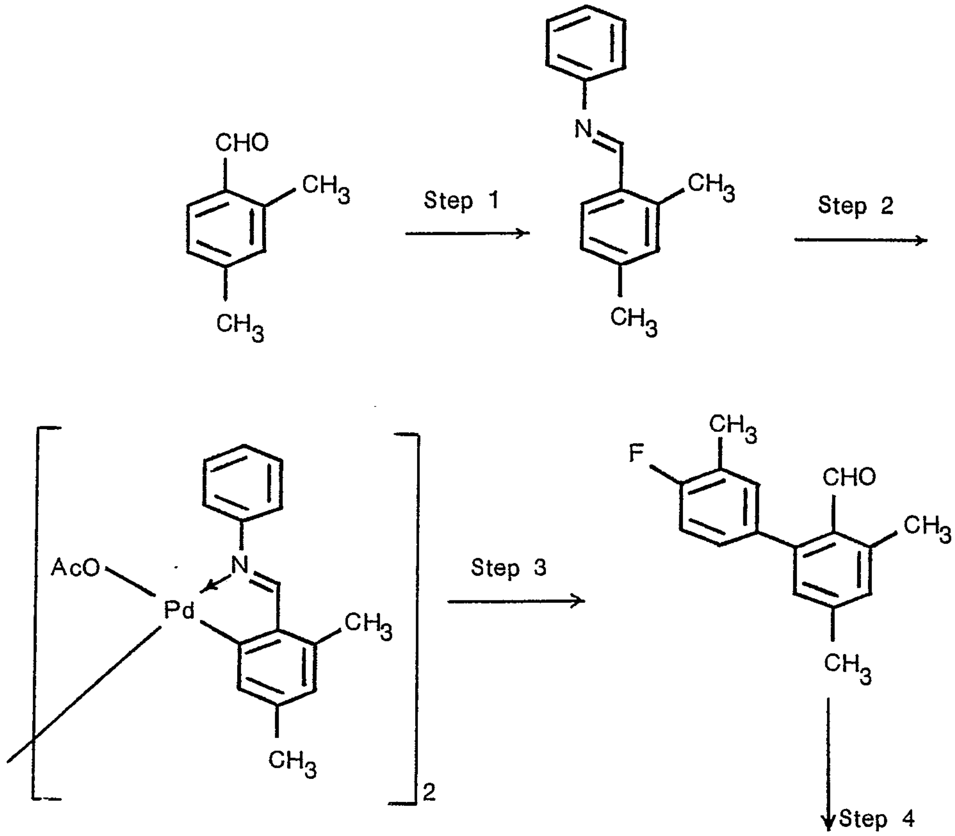
Step 1
Step 2
Step 3
Step 4

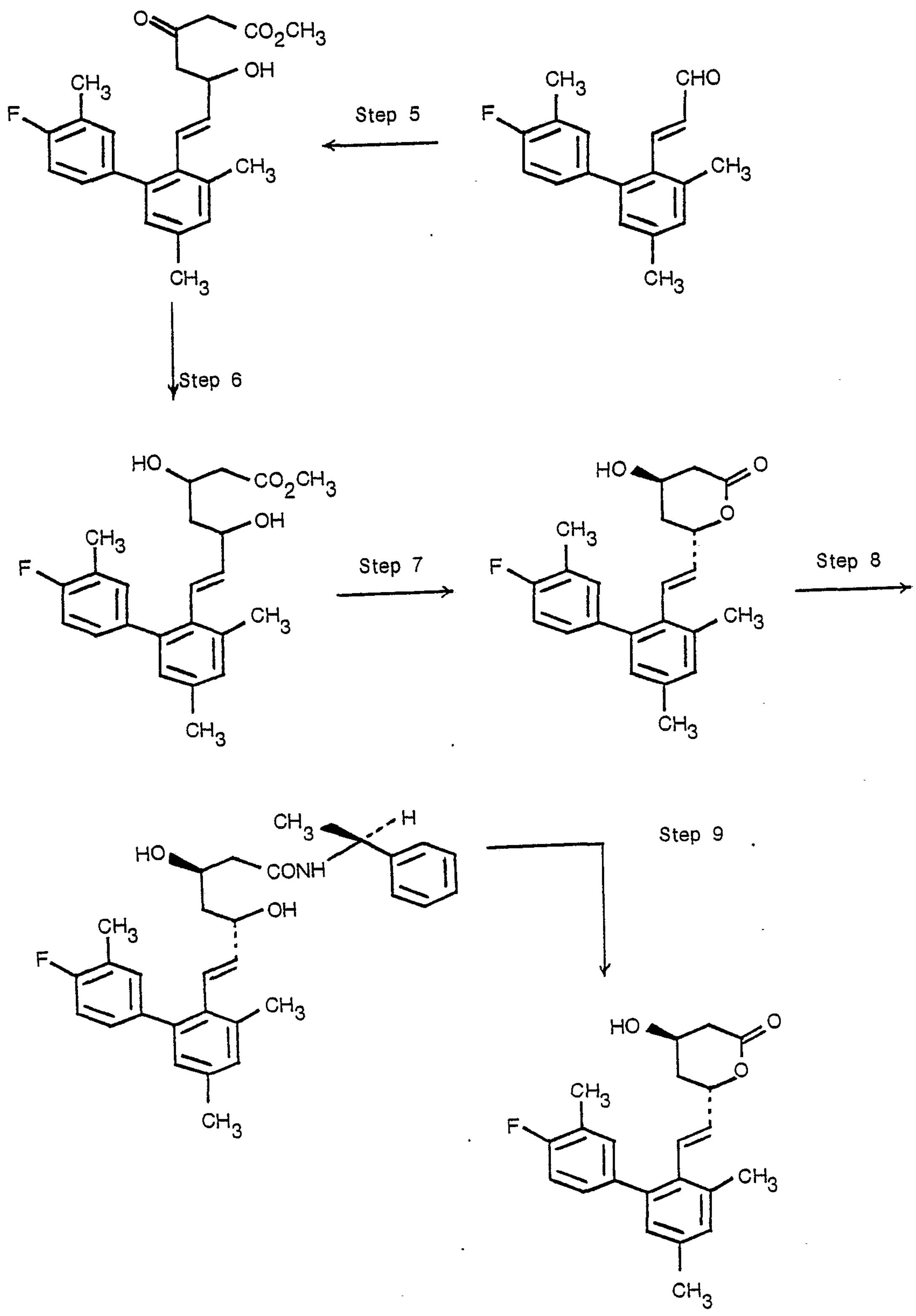


III

Reactions in the flow sheet

1. Reaction with aniline in refluxing toluene.
2. Reaction with palladium (II) acetate in acetic acid at reflux.
3. Reaction with a substituted Grignard reagent

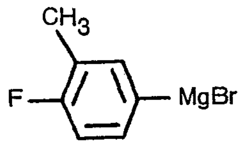

in a suitable solvent such as benzene or toluene in the presence of triphenylphoshine followed by hydrolysis with 6N HCl at ambient temperature.

4. Aldol Reaction.

a) The classical Aldol synthesis in which acetaldehyde is condensed with the starting benzaldehyde, the resulting β-hydroxyaldehyde is acetylated with acetic anhydride and acetic acid is eliminated thermally to give the corresponding cinnamaldehyde.

b) The directed Aldol condensation in which the anion of an appropriately N-substituted ethylidenylimine, such as ethylidenecyclohexylimine, is condensed with the starting benzaldehyde at or below room temperature in an aprotic solvent, such as THF, to afford a β-hydroxy-β-phenylpropylidenylimine which, upon concomitant dehydration and imine hydrolysis in an acidic medium, such as dilute aqueous HCl, provides the corresponding cinnamaldehyde.

c) The use of a nucleophilic acetaldehyde equivalent in which *cis*-2-ethoxyvinyllithium, generated from *cis*-1-ethoxy-2-tri-*n*-butylstannylethylene, is condensed with the starting benzaldehyde to give an allylic alcohol which is subsequently rearranged, under suitable acidic conditions, to the corresponding cinnamaldehyde.

5. Dianion Step. Reaction with the dianion of acetoacetic acid methyl ester e.g. in a suitable aprotic solvent such as THF and dioxane.

6. Reduction e.g. with $NaBH_4$ in a suitable solvent such as methanol and ethanol at or below room temperature.

7. Lactonization. Saponification by base (e.g. NaOH) e.g. in aqueous alcohol followed by lactonization, e.g. by acidification and cyclodehydration by heating in toluene followed by separation of the *cis* and *trans* mixture e.g. by chromatography on silica gel or crystallization.

8. Resolution of the *trans* racemate into its enantiomers by treating the (±)-*trans* lactone with either *d*-(+) or *l*-(−)-α-methylbenzylamine to give the diastereomeric dihydroxy amides which are separated e.g. by chromatography or crystallization.

9. Hydrolysis of each pure diastereomeric amide e.g. under basic conditions, such as ethanolic NaOH to afford the corresponding enantiomerically pure dihydroxy acid which, upon lactonization, e.g., in refluxing toluene, provides the pure (+)-*trans* enantiomer. Stereochemistry depends on the absolute stereochemistry of the diastereomeric amide from which it is derived.

A further aspect of the present invention is a pharmaceutical composition consisting of compound III in association with a pharmaceutical vehicle or diluent. The pharmaceutical composition can be formulated in a classical manner utilizing solid or liquid vehicles or diluents and pharmaceutical additives of a type appropriate to the mode of desired administration. The compounds can be administered by an oral route, for example, in the form of tablets, capsules, granules or powders, or they can be administered by a parenteral route in the form of injectable preparations. The dose to be administered depends on the unitary dose, the symptons, and the age and the body weight of the patient. A dose for adults is preferably between 200 and 2,000 mg per day, which can be administered in a single dose or in the form of individual doses from 1—4 times per day.

A typical capsule for oral administration contains active ingredient (250 mg), lactose (75 mg) and magnesium stearate (15 mg). The mixture is passed through a sieve (sieve opening: 250 μm — 60 mesh) and packed into a No. 1 gelatin capsule.

A typical injectible preparation is produced by asceptically placing 250 mg of sterile active ingredient into a vial, asceptically freeze-drying and sealing. For use, the contents of the vial are mixed with 2 ml of physiological saline, to produce an injectible preparation.

The compound of this invention also has useful antifungal activity. For example, it may be used to control strains of *Penicilium sp., Aspergillus niger, Cladosporium sp., Cochliobolus miyabeonus* and *Hilminthosporium cynodnotis.* For this utility it is admixed with suitable formulating agents, powders, emulsifying agents or solvents such as aqueous ethanol and sprayed or dusted on the plants to be protected.

This invention can be illustrated by the following examples in which ratios of solvents are in volumes and percentages, unless otherwise indicated, or by weight.

Example

(+)-(4R,6S)-(E)-6-[2-(4′-fluoro-3,3′,5-trimethyl[1,1′-biphenyl]-2-yl)-ethenyl]-3,4,5,6-tetrahydro-4-hydroxy-2H-pyran-2-one

Step 1: Preparation of N-[(2,4-Dimethylphenyl)-methylene]benzeneamine

A mixture of 2,4-dimethylbenzaldehyde (53.7 g, 0.4 mole), freshly distilled aniline (37.5 g, 0.4 mole) and toluene (150 ml) was heated at reflux under a Dean-Stark trap for 2 hours. The solution was cooled and then

concentrated under reduced pressure. Distillation of the residue at 0.2 mm gave the product as a yellow oil (81.3 g, 97% yield), bp 122—130°, which solidified on cooling.

Step 2: Prepartion of Bis(μ-(Acetato-0:0')bis(3,5-dimethyl-2-[(phenylimino)methyl]phenyl-C,N]-dipalladium

A mixture of N-[(2,4-dimethylphenyl)-methylene]benzeneamine (58.7 g, 0.28 mole) and palladium (II) acetate (62.9 g, 0.28 mole) in acetic acid (1 L) was heated at reflux with stirring for one hour. After cooling (to 50°), the reaction mixture was filtered (gravity) and the filtrate poured into water (4 L). The aqueous mixture was stirred overnight and then filtered. The collected solid was washed with several portions of cold water, air dried for several hours (with suction) and dried in a vacuum oven (45°) for 2 days to afford the desired product as a yellow-orange solid (101.3 g, 97% yield).

Step 3: Preparation of 4'-Fluoro-3,3',5-trimethyl-1,1'-biphenyl-2-carboxaldehyde

In a 1 L, 3-neck flask equipped with a magnetic stirring bar, $N_2$ inlet tube, dropping funnel (250 ml), and reflux condenser capped with a drying tube, was placed Mg turnings 4.5 g (0.185 mole). This equipment was heated with a heat gun under $N_2$ and then allowed to cool to room temperature. 5-Bromo-2-fluorotoluene (35.0 g, 0.185 mole) was dissolved in 300 ml of ether and 40 ml of this solution was added to the Mg turnings. After mild heating with a heat gun to initiate the reaction, the remainder of the ether solution was added at such a rate as to maintain reflux (one hour). The mixture was stirred at reflux for an additional 30 minutes and cooled to room temperature.

Meanwhile, the Pd complex (45.2 g, 0.06 mole) from Step 2 had been added to 1 L of toluene (3 L, 3-neck flask). The resulting mixture was stirred vigorously and heated at reflux for one hour using a Dean-Stark trap to collect traces of water. The mixture was cooled to room temperature under $N_2$, triphenylphosphine (64.0 g, 0.24 mole) was added and the mixture was stirred for 30 minutes. The Grignard reagent was added by means of a dropping funnel in a slow, steady stream and the reaction mixture stirred for one hour. After addition of 200 ml 6N HCl, the mixture was stirred for an additional one hour and filtered. The collected solid was washed with several portions of toluene. The filtrate and washings were combined and separated. The toluene-ether solution was washed with 2 × 200 ml of brine and dried over $MgSO_4$. Filtration and evaporation yielded a black oil (70 g) which was chromatographed using a 120 mm "Still" column (1.5 kg of 37—62 μm Silica Gel) and 40% (v:v) $CH_2Cl_2$-hexane. There were collected 2 fractions of 1000 ml and 20 fractions of 5000 ml, the pure product being found in fractions 5—14. Fractions 14—17 contained a mixture of the desired aldehyde plus 2,4-dimethylbenzaldehyde. There was obtained 19—21 g of combined product as a pale yellow solid (70% yield); one spot on tlc ($R_f$=0.30 on Silica Gel GF plate with 40% (v:v) $CH_2Cl_2$-hexane), mp 75—78°C. The mp of pure sublimed product is 78—80°.

Step 4:

Preparation of 3-(4'-Fluoro-3,3',5-trimethyl-[1,1'-biphenyl]-2-yl)-2-propenal

A dry 1 L, 4-neck flask equipped with a magnetic stirring bar, $N_2$ inlet tube, thermometer, addition funnel (capped with a drying tube) and septum was charged with a solution of (Z)-1-ethoxy-2-tributylstannylethylene (68.5 g, 0.19 mole) in dry THF (200 ml) and then cooled in a dry ice-acetone bath. n-Butyllithium (1.48 M in hexane, 130 ml, 0.19 mole) was added via syringe over a period of 25 minutes. The resulting mixture was stirred at −75° under $N_2$ for one hour. A solution of 4'-fluoro-3,3',5-trimethyl-1,1'-biphenyl-2-carboxaldehyde (40.7 g, 0.168 mole) in THF (150 ml) was added dropwise (one hour) at −75°. After stirring for 10 minutes, the cooling bath was removed and the reaction mixture was stirred at ambient temperature for 1—1/2 hours. Saturated aq. $NaHCO_3$ (150 ml) was added in a slow steady stream and the mixture, distributed between ether (500 ml) and water (500 ml). After separating the layers, the aqueous phase was extracted with ether (2x). The ether extracts were combined, washed with cold water and brine, dried over $MgSO_4$, filtered and evaporated.

The residual oil was taken up in THF (200 ml) and the solution treated with 6N HCl (25 ml) and stirred at room temperature for one hour. The mixture was diluted with cold water and extracted (3x) with ether. The ether extracts were combined, washed with cold water and brine, and dried over $MgSO_4$. Filtration and evaporation yielded a yellow brown oil (120 g), which was chromatographed using 2.6 kg of Silica Gel (37—62 μm) in a 140 mm "Still" column. Elution with $CH_2Cl_2$-hexane (3:1, v:v) gave 21 fractions (1 L each), the major portion of product being location in fractions 11—19. After evaporation, the oil was taken up in warm hexane (60 ml); the solution was seeded and cooled at room temperature to give a white crystalline solid (20.6 g), mp 82—85°. Fractions 9 and 10 were handled separately and provided after recrystallization from hexane an additional 3.6 g of product; total quantity was 24.2 g (54% yield).

Step 5: Preparation of Methyl (E)-7-(4'-fluoro-3,3',5-trimethyl[1,1'-biphenyl]-2-yl)-5-hydroxy-3-oxo-6-heptenoate

Methyl acetoacetate (12.31 g, 0.106 mole) was added dropwise to a stirred suspension of sodium hydride (50% oil suspension) (5.09 g, 0.106 mole) in dry THF (180 ml) at 0° (internal, keeping temperature 10°C) and under $N_2$. The resulting solution was stirred at 0° for 15 minutes and then treated with a solution of 1.40 M n-butyllithium in hexane (78.7 ml, 0.106 mole) added dropwise (20 minutes) via syringe. After stirring at 0° for 20 minutes, the bath was replaced by an ice-acetone bath and stirred for an additional 5 minutes. A solution of 3-(4'-fluoro-3,3',5-trimethyl[1,1'-biphenyl]-2-yl)-2-propenal (26.5 g, 0.099 mole) in

dry THF (150 ml) was added dropwise (15 minutes) at such a rate as to keep the temperature 10°C. The reaction mixture was stirred at 0°C for 30 minutes and then quenched by the slow addition of 6N HCl (45 ml). The mixture was then diluted with water (300 ml) and extracted (3x) with ether. The ether extracts were combined, washed with cold water and brine, dried ($MgSO_4$), filtered and evaporated leaving a yellow oil (38 g); one major spot on tlc (Silica Gel GF) at $R_f$ = 0.49 (5% (v:v) acetone—$CH_2Cl_2$).

Step 6: Preparation of Methyl (E)-7-(4′-fluoro-3,3′,5-trimethyl[1,1′-biphenyl)-2-yl)-3,5-dihydroxy-6-heptenoate

Sodium tetrahydridoborate (1.92 g, 0.0507 mole) was added portionwise over a period of 10 minutes and with stirring to a cold solution (0°C) of methyl (E)-7-(4′-fluoro-3,3′,5-trimethyl[1,1′-biphenyl]-2-yl)-5-hydroxy-3-oxo-6-heptenoate (37.5 g) in methanol (400 ml). The clear reaction mixture was stirred at 0°C for 15 minutes and then diluted with water (300 ml). While maintaining the temperature below 10°C with an ice-bath, the mixture was acidified with 12N HCl (22 ml). Following extraction with ether (3 × 250 ml), the ether extracts were combined, washed with cold water and brine, dried ($MgSO_4$) and evaporated to a viscous oil (38 g). The product showed one major spot on tlc (silica gel GF) with $R_f$ 0.21 (10% (v:v) acetone—$CH_2Cl_2$).

Step 7: Preparation of (±) trans (E)-6-[2-(4′-Fluoro-3,3′,5-trimethyl[1,1′-biphenyl]-2-yl)-ethenyl]-3,4,5,6-tetrahydro-4-hydroxy-2H-pyran-2-one

A solution of methyl (E)-7-(3′-fluoro-3,3′,5-trimethyl[1,1′-biphenyl]-2-yl)-3,5-dihydroxy-6-heptenoate (37.7 g, .0976 mole) and 1N sodium hydroxide (100 ml, 0.10 mole) in methanol (150 ml) was stirred at room temperature for 15 minutes. After removal of the methanol by evaporation (30°), the reaction mixture was diluted with water (600 ml), acidified with conc. HCl and extracted with ether (3 × 300 ml). The ether extracts were combined, washed with cold water and brine, dried over $MgSO_4$, filtered, and evaporated to yield a yellow-orange oil (37 g).

A solution of the crude oily diol acid in toluene (300 ml) was heated at reflux under a Dean-Stark trap for one hour. Evaporation provided a yellow oil (37 g) which was a mixture of *cis* and *trans* lactones. The crude product was chromatographed using a 140 mm "Still" column (37—62, µm Silica Gel) and 10% (v:v) acetone—$CH_2Cl_2$. After collecting 3 fractions of 1L and 25 fractions of 500 ml, elution was continued with 20% (v:v) acetone $CH_2Cl_2$ collecting 6 fractions of 1 L each, containing primarily *cis* racemate (84%). Fractions 12—15 of the 500 ml cuts were combined and evaporated to give the *trans* racemate as a pale yellow oil (90% pure by HPLC). This was crystallized from $Et_2O$-hexane to give a solid (5 g) mp 115—117°. Fractions 16—25 were combined and evaporated to give a pale yellow oil (12 g), which was a (6:4) mixture of *trans* and *cis* racemates.

This mixture (12 g) was re-chromatographed in two runs with a Waters Prep LC500, employing 2 prep PAK-500 silica cartridges in series and eluting with acetone—$CH_2Cl_2$ (1:9, v:v). Using the shave recycle technique, the *trans* isomer (5 g) and the *cis* racemate (4 g) were obtained. The samples of the *trans* racemate, collected from the two chromatographic separations, were combined to give a white crystalline solid (10 g) mp 115—117°. All *cis* fractions from chromatography and the Waters separation were combined to give 9 g which crystallized on standing.

Step 8: Preparation of 3R,5S)-N-((S)-α-Methylbenzyl)-7-(4′-fluoro-3,3′,5-trimethyl[1,1′-biphenyl]-2-yl)-3,5-dihydroxy-6-heptenamide

A solution of (±)-*trans*-(E)-6-[2-(4′-fluoro-3,3′,5-trimethyl[1,1′-biphenyl]-2-yl)ethenyl]-3,4,5,6-tetrahydro-4-hydroxy-2H-pyran-2-one (10 g, 28.2 mole) and (S)-(−)-α-methylbenzylamine (36.35 ml, 34.17 g, 282 mmole) in THF (35 ml) was stirred at gentle reflux under $N_2$ for 16 h. The reaction mixture was then cooled, diluted with $Et_2O$ (400 ml) and successively washed with $H_2O$ (2 × 200 ml), 3N HCl (2 × 300 ml) and brine (2 × 200 ml), dried ($MgSO_4$) and filtered. The filtrate was evaporated leaving the mixture of intermediate diastereomeric amides as a pale amber gum.

The residual gum was digested in $Et_2O$ (100 ml) and treated with hexane until turbid. This was seeded and cooled at 0°. Solid, 1.5 g, m.p. 110—12°, was collected by filtration. The filtrate was evaporated to a gum (12 g).

This mixture of diastereomeric amides was chromatographed using a Waters Prep LC500. Separation of the mixture was accomplished using two prep PAK-500/silica cartridges in series eluting with methylene chloride-acetone (80:20, v:v). The mixture was divided and run in two separate elutions. Using the shave recycle technique, the two diastereoisomers (1) (4.5 g, m.p. 110—12°) and (2) (3.5 g, 52.6% of theory) m.p. 86—9° were obtained. Compound (2) which was 99% pure by HPLC is the desired diastereoisomer. Mother liquors from recrystallization of (2) were combined (1.9 g, 85% (2)).

Step 9: Preparation of (+)-(4R,6S)-(E)-6-[2-(4′-Fluoro-3,3′,5-trimethyl[1,1′-biphenyl]-2-yl)-ethenyl]-3,4,5,6-tetrahydro-4-hydroxy-2H-pyran-2-one.

A solution of N-((S)-α-methylbenzyl)-7-(4′-fluoro-3,3′,5-trimethyl[1,1′-biphenyl]-2-yl)-3,5-dihydroxy-6-heptenamide (3.5 g, 7.35 mmol), diastereoisomer (2) in step 8 above, in EtOH (300 ml) containing 1N NaOH (44 ml, 44 mmole) and $H_2O$ (44 ml) was refluxed under $N_2$ for 14 h. The solvent was removed *in vacuo* (40°). The residue was dissolved in ice water (300 ml) and $Et_2O$ (500 ml), then cooled and stirred at 0° while 3N HCl

(50 ml) was slowly added. The organic layer was separated and washed successively with ice-cold 1N HCl (200 ml) and brine (2 × 200 ml), dried ($MgSO_4$) and filtered. After evaporation of solvent *in vacuo,* the remaining residue was dissolved in toluene (500 ml) and refluxed under a Dean-Stark trap for 2h. Evaporation provided a yellow oil which was chromatographed on a 50 mm "Still" column (37—62 µm Silica Gel) using acetone-methylene chloride (10:90, v:v) and collecting 10 ml fractions. The product was found in cuts 19—31 followed by cuts slightly contaminated with cis lactone. Less than pure fractions were combined and, after evaporation, further enriched by crystallization from $Et_2O$-hexane. The combined yield was 1.6 g, 61%. This was 99.99% pure by HPLC, m.p. 87—9°. $[\alpha]_D^{25}$ + 40.56° (C = 1.075, $CHCl_3$).

**Claims for the Contracting States: BE CH DE FR GB IT LI LU NL SE**

1. A process for the preparation of (+)-(4R,6S)-(E)-6-[2-(4'-fluoro-3,3',5-trimethyl[1,1'-biphenyl]-2-yl)ethenyl]-3,4,5,6-tetrahydro-4-hydroxy-2H-pyran-2-one characterized in that:

a) condensation of the dianion of acetoacetic acid methyl ester with an aldehyde of structure:

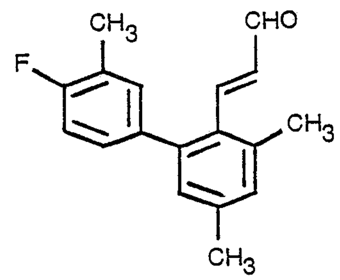

to give structure:

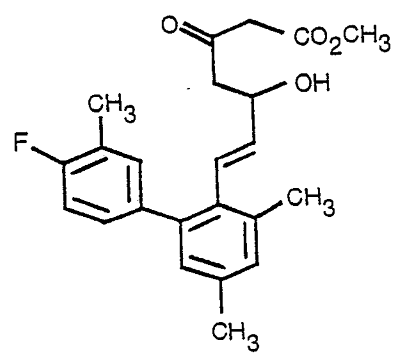

b) reduction of the keto group to an hydroxyl;

c) saponification of the ester, lactonization and separation of the *cis* and *trans* racemates;

d) formation of the diastereomeric α-methyl benzylamides of the *trans*-racemate and separation of the diastereomers; and

e) hydrolysis of the diastereomer containing the product (+)-*trans* enantiomer and lactonization.

2. The compound, (+)-(4R,6S)-(E)-6-[2-(4'-fluoro-3,3',5-trimethyl[1,1'-biphenyl]-2-yl)-ethenyl)-3,4,5,6-tetrahydro-4-hydroxy-2H-pyran-2-one, the corresponding dihydroxy acid, or a pharmaceutically acceptable salt thereof.

3. A pharmaceutical composition comprising a pharmaceutical carrier and an effective amount of (+)-(4R,6S)-(E)-6-[2-(4'-fluoro-3,3',5-trimethyl[1,1'-biphenyl]-2-yl)ethenyl]-3,4,5,6-tetrahydro-4-hydroxy-2H-pyran-2-one, the corresponding dihydroxy acid or a pharmaceutically acceptable salt thereof.

**Claim for the Contracting State AT:**

1. A process for the preparation of (+)-(4R,6S)-(E)-6-[2-(4'-fluoro-3,3',5-trimethyl[1,1'-biphenyl]-2-yl)-ethenyl]-3,4,5,6-tetrahydro-4-hydroxy-2H-pyran-2-one characterized in that:

a) condensation of the dianion of acetoacetic acid methylester with an aldehyde of structure:

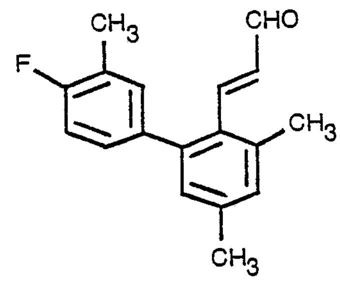

to give structure:

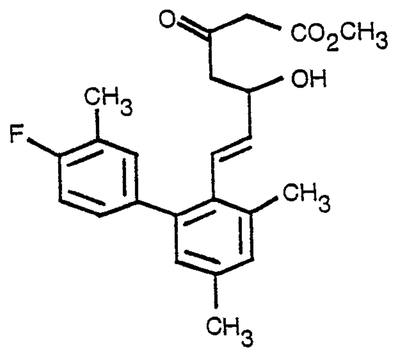

b) reduction of the keto group to an hydroxyl;
c) saponification of the ester, lactonization and separation of the *cis* and *trans* racemates;
d) formation of the diastereomeric α-methyl benzylamides of the *trans*-racemate and separation of the diastereomers; and
e) hydrolysis of the diastereomer containing the product (±)-*trans* enantiomer and lactonization.

**Revendications pour les Etats contractants: BE CH DE FR GB IT LI LU NL SE**

1. Un procédé pour la préparation de la (+)-(4R,6S)-(E)-6-[2-(4'-fluoro-3,3',5-triméthyl[1,1-biphényl)-2-yl)éthényl]-3,4,5,6-tetrahydro-4-hydroxy-2H-pyranne-2-one caractérisé par:
a) la condensation du dianion d'un méthylester d'acide acétoacétique avec un aldéhyde de structure:

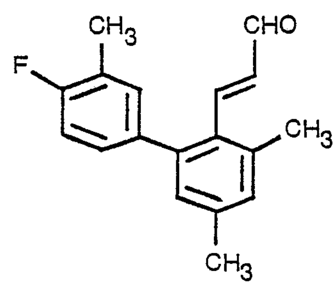

pour obtenir la structure:

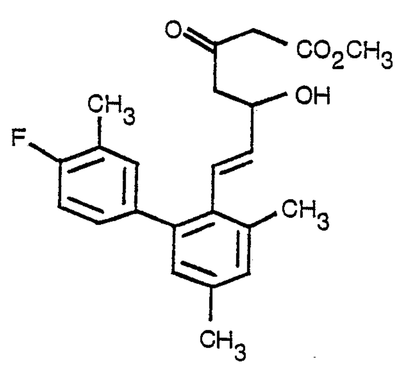

b) la réduction du groupe céto en un hydroxyle;

c) la saponification de l'ester, la lactonisation et la séparation des Racé mates *cis* et *trans*;

d) la formation des α-méthylbenzylamides diastéréo-siomères du racémate *trans* et la séparation des diastéréo-isomères; et

e) l'hydrolyse du diastéréo-isomère contenant l'énantiomère (±)-*trans* produit et la lactonisation.

2. Le composé (+)-(4R,6S)-(E)-6-[2-(4'-fluoro-3,3',5-triméthyl[1,1'-biphenyl]-2-yl)éthényl]-3,4,5,6-tétrahydro-4-hydroxy-2H-pyranne-2-one, le dihydroxyacide correspondant ou un sel acceptable en pharmacie correspondant.

3. Une composition pharmaceutique comprenant un support pharmaceutique et une quantité efficace de (+)-(4R,6S)-(E)-6-[2-(4'-fluoro-3,3',5-triméthyl[1,1'-biphényl]-2-yl)éthényl]-3,4,5,6-tétrahydro-4-hydroxy-2H-pyranne-2-one, du dihydroxyacide correspondant ou d'un sel acceptable en pharmacie correspondant.

**Revendication pour l'Etat contractant: AT**

1. Un procédé pour la préparation de la (+)-(4R,6S)-(E)-6-[2-(4'-fluoro-3,3',5-triméthyl[1,1'-biphényl]-2-yl)éthényl]-3,4,5,6-tétrahydro-4-hydroxy-2H-pyranne-2-one caractérisé par:

a) la condensation du dianion d'un méthylester d'acide acétoacétique avec un aldéhyde de structure:

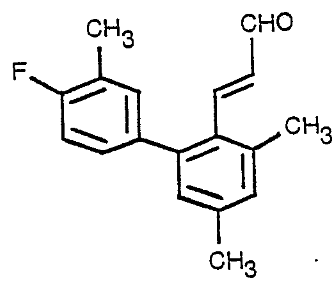

pour obtenir la structure:

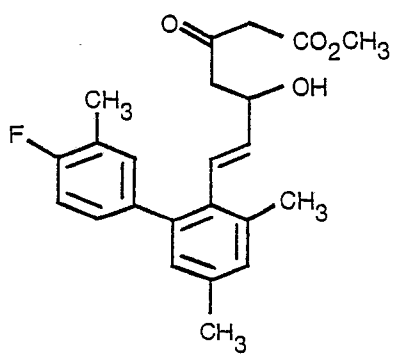

b) la réduction du groupe céto en un hydroxyle;

c) la saponification de l'ester, la lactonisation et la séparation des racémates *cis* et *trans*;

d) la formation des α-méthylbenzylamides diastéréo-isomères du racémate *trans* et la séparation des diastéréo-isomères; et

e) l'hydrolyse du diastéréo-isomère contenant l'énantiomère (±)-*trans* produit et la lactonisation.

**Patentansprüche fur die Vertragsstaaten: BE CH DE FR GB IT LI LU NL SE**

1. Ein Verfahren zur Herstellung von (+)-(4R,6S)-(E)-6-[2-(4'-Fluor-3,3',5-trimethyl[1,1'-biphenyl]-2-yl)-ethenyl]-3,4,5,6-tetrahydro-4-hydroxy-2H-pyran-2-on, dadurch gekennzeichnet, daß:

a) das Dianion des Acetessigsäuremethylesters mit einem Aldehyd der Struktur:

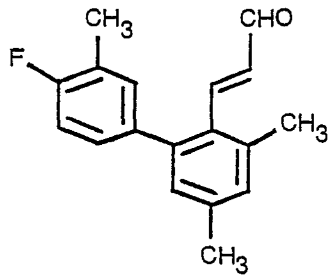

unter Bildung der Struktur:

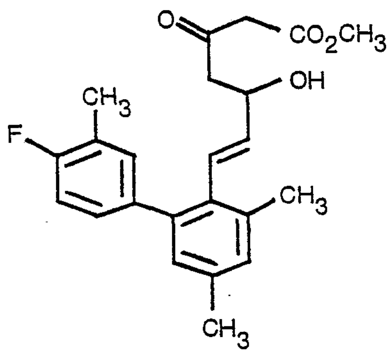

kondensiert wird;

b) die Ketogruppe zu einer Hydroxylgruppe reduziert wird;

c) der Ester verseift und lactonisiert wird, und die cis- und trans-Racemate getrennt werden;

d) die diastereomeren α-Methylbenzylamide des trans- Racemats gebildet und die Diastereomeren getrennt werden; und

e) das das Produkt (+)-trans-Enantiomer enthaltende Diastereomer hydrolysiert und lactonisiert wird.

2. Die Verbindung (+)-(4R,6S)-(E)-6-[2-(4'-Fluor-3,3',5-trimethyl[1,1'-biphenyl]-2-yl)-ethenyl]-3,4,5,6-tetrahydro-4-hydroxy-2H-pyran-2-on, die entsprechende Dihydroxysäure oder ein pharmazeutisch annehmbares Salz davon.

3. Eine pharmazeutische Zusammensetzung, enthaltend einen pharmazeutischen Träger und eine wirksame Menge von (+)-(4R,6S)-(E)-6-[2-(4'-Fluor-3,3',5-trimethyl[1,1'-biphenyl]-2-yl)ethenyl]-3,4,5,6-tetrahydro-4-hydroxy-2H-pyran-2-on, der entsprechenden Dihydroxysäure oder eines pharmazeutisch annehmbaren Salzes davon.

**Patentanspruch fur die Vertragsstaat: AT**

1. Ein Verfahren zur Herstellung von (+)-(4R,6S)-(E)-6-[2-(4'-Fluor-3,3',5-trimethyl[1,1'-biphenyl]-2-yl)-ethenyl]-3,4,5,6-tetrahydro-4-hydroxy-2H-pyran-2-on, dadurch gekennzeichnet, daß:

a) das Dianion des Acetessigsäuremethylester mit einem Aldehyd der Struktur:

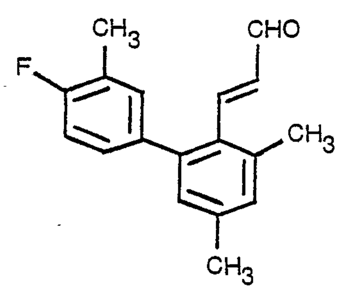

unter Bildung der Struktur:

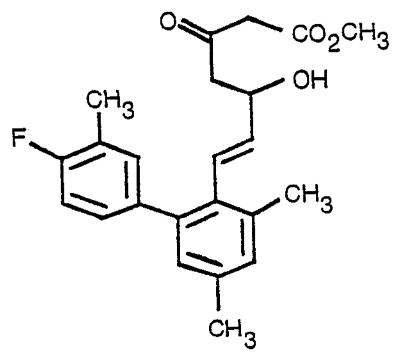

kondensiert wird;

b) die Ketogruppe zu einer Hydroxylgruppe reduziert wird;

c) der Ester verseift und lactonisiert wird, und die cis- und trans-Racemate getrennt werden;

d) die diastereomeren α-Methylbenzylamide des trans-Racemats gebildet und die Diastereomeren getrennt werden; und

e) das das Produkt (+)-trans-Enantiomer enthaltende Diastereomer hydrolysiert und lactonisiert wird.